# EUROPEAN PATENT APPLICATION

(11) **EP 4 311 548 A1**
(43) Date of publication of application: **31.01.2024**
(21) Application number: 22306143.3
(22) Date of filing: 29.07.2022
(51) Int. Cl.: A61K 31/196, A61K 31/4184, A61K 45/06, A61P 35/00, A61P 35/04

(54) **COMBINATION OF A NKCC1 PROTEIN INHIBITOR AND A MICROTUBULE INHIBITOR FOR USE IN THE TREATMENT OF CANCER**

(71) Applicant: B & A Therapeutics, 13288 Marseille Cedex 09 (FR)
(72) Inventor: BEN-ARI, Yéhézkel, 13600 La Ciotat (FR); BERGER, François, 28240 MEYLAN (FR)
(74) Representative: Mundel, Lysiane

(57) **Abstract**

The present invention relates to a combination of compounds for a use in therapy and especially to a therapeutic combination for use int the treatment of tumours and/or cancer. In particular, the invention relates to a therapeutic combination that comprises a NKCC1 protein inhibitor and a microtubule inhibitor, optionally together with one or more additional chemotherapeutic agents or radiotherapy. The combined administration includes at least one NKCC1 protein inhibitor and at least one microtubule inhibitor.

## Description

The present invention relates to a combination of compounds for a use in therapy and especially to a therapeutic combination useful for the treatment of cancer. In particular, the invention relates to a therapeutic combination that comprises a NKCC1 protein inhibitor and a compound able to destabilize the tubulin polymerization, optionally together with one or more additional chemotherapeutic agents or radiotherapy. The combined administration includes at least one NKCC1 protein inhibitor and at least one microtubule inhibitor.

The present invention is based on the combination of compounds having different ways of action, for a synergistic effect in therapy and especially in cancer therapy. The targeted mechanisms are the NKCC1 protein inhibition and the tubulin polymerization inhibition in cells cytoskeleton. NKCC1 protein inhibitors are considered as attractive drug targets in central nervous system but they have also showed interesting effects in other therapies such as cancer therapy. Considering bumetanide as the representative of the NKCC1 protein inhibitors, the drawbacks of its use as a drug are its poor brain bioavailability, its effects on kidneys, and its ototoxicity especially in paediatric populations. The chronic use of a NKCC1 inhibitor can be problematic. The microtubule inhibitors are known as microtubule-targeting drugs and are already in clinical use for the treatment of cancers. They are able to disrupt the microtubules and inhibit mitosis; cancer cells replicate rapidly so they are constantly undergoing mitosis. Unfortunately their use has several drawbacks such as acquired resistance and severe side effects (gastrointestinal problems, hair loss, alopecia, neurotoxicity, autonomic system malfunction, etc.).

The present invention is based on the above detailed targeted mechanisms of NKCC1 inhibition and microtubule inhibition at the same time for a use in therapy. The main objective is to reduce the doses of each compound because of a synergistic effect of said approach. Bumetanide is the best-known representative of the NKCC1 inhibitors. The molecular identification of NKCC1 and NKCC2 was made in 1994 (Delpire et al. 1994; Gamba et al., 1994 et Xu et al.) after the clinical approval of bumetanide as a drug in the treatment of hypertension and brain edema. In the 1960s, the exact target of bumetanide in the kidney was not known. Bumetanide was discovered as a result of systematic structure-activity studies of a large series of diuretically active 3-amino-5-sulfamoylbenzoic acid derivatives (Felt 1981) and was followed by several series of compounds related both in chemical structure and diuretic profile. In other words, the synthesis of sulfamoyl benzoic acid diuretics was done long before their cellular and molecular mechanisms of action were elucidated.

Bumetanide has been marketed in more than fifty countries including Germany, Japan, Denmark, France, Sweden, the Netherlands, New Zealand, Belgium, Finland, Russia and the United States of America for the treatment of oedema associated with congestive heart failure, hepatic disease and renal disease.

It was shown that NKCC1 inhibition by the highly specific inhibitor bumetanide, rescues GABA transmission and behavioural deficits in animal models and in patients. Compelling evidence including clinical trials have been made for Autism Spectrum Disorders (ASD) and Parkinson Disease (PD)(Savardi et al., Trends Pharmacol. Sci., 2021; Yehezkel Ben-Ari, Trends Neurosc., 2017; Hadjikhani et al., Autism Res., 2017; Damier et al., Clin. Neuropharmacol., 2016) or Down syndrome (Deidda et al., Nat. Med., 2015). Another recent study raises the possibility that Bumetanide might also be efficient to treat Alzheimer Disease since use of Bumetanide in elderly (more than 65 years old) decreases the incidence of Alzheimer by 30% to 70% and Bumetanide is the best of 1300 widely used agents to attenuates Alzheimer signatures in vitro (Taubes et al., Am. J. Clin. Nutr., 2021).

The present invention is based on the use of specific inhibitors of NKCC1, a cation-chloride cotransporter (CCCs) in the treatment of tumours and cancer. CCCs are secondary active symporters that control the K⁺ and/or Na⁺ gradients to extrude or uptake Cl⁻ in various cell types. NKCC1 is an intensively studied member of the CCC family and plays fundamental roles in regulating trans-epithelial ion movement, cell volume, chloride homeostasis and neuronal excitability.

NKCC symporters are found with two subtypes, ubiquitous NKCC1 and kidney-specific NKCC2. The most potent inhibitor of NKCC1 and NKCC2 is Bumetanide, a loop diuretic. Bumetanide (known as Burinex^{®}) is indicated for the treatment of oedema associated with congestive heart failure, cirrhosis of the liver and renal disease including the nephrotic syndrome. Bumetanide acts on the thick ascending limb of the loop of Henle, where it can block the luminal Na-K-2Cl (NKCC2) transporter, a protein that transports sodium, potassium, and chloride from pro-urine to interstitial fluid/blood, thereby increasing urinary loss of water, sodium, potassium, and chloride. The NKCC1 chloride co-transporter regulates in virtually all cells the influx of ions notably chloride and when excessively operating leads to chloride intracellular accumulation.

Alterations in the expression and function of NKCC1 have been implicated in several brain and peripheral disorders, including neonatal seizures, epilepsy, autism, cerebral oedema following ischemic and traumatic brain injury, chronic, spinal cord lesions, chronic pain, Alzheimer disease and acute pain but also various types of cancer and brain tumours (Yehezkel Ben-Ari 2017 ; Savardi et al. 2021). Excessive activity of NKCC1 in neurons leads to a shift of the polarity of GABA and a loss of the main inhibitory drive in the brain but also swelling consequently to a disturbance of sodium and chloride leading to water influx. This in turn impacts brain inhibitory networks & behaviourally relevant oscillations. Depolarizing /excitatory actions of GABA have been reported in these disorders often associated with a hyperphosphorylation of NKCC1 and a loss of activity of the main chloride exporter KCC2. Indeed, the latter is instrumental in removing chloride and highly vulnerable to insults that lead to its internalization. The combined overactivity of NKCC1 and reduced activity of KCC2 underlie the chloride accumulation and the loss of inhibitory drive (Yehezkel Ben-Ari 2015; Y. Ben-Ari et al. 2007; Yehezkel Ben-Ari 2017; Glykys et al. 2017; Dzhala et al. 2010). This universal reaction constitutes a final common neuronal response to many brain disorders in spite of their heterogeneity. Collectively, the data available suggests that a selective agent like Bumetanide might provide a treatment to many central and peripheral brain disorders.

Extensive investigations suggest that the electroneutral NKCC1 cotransporter plays an important role in many brain disorders diseases and symptoms. These effects are mediated by an overactivity of NKCC1 leading to major ionic changes and alterations of neuronal volume associated with swelling that in turn impacts essential functions including migration, movement and apoptosis. Earlier studies conducted by several groups have shown that a similar situation occurs in glioblastoma with bumetanide attenuating metastases and migration from the tumours in vitro and in vivo (Luo et al., 2020; Xu et al., 2016; Hu et al., 2019). Among the compounds known to destabilize the tubulin polymerization, we can cite the benzimidazole anthelmintics, the taxanes, the vinca alkaloids and the epothilones.

The best-known members of the benzimidazole anthelmintics are mebendazole (MBZ) and albendazole (ABZ). Mebendazole, methyl N-[6-(benzoyl)-1H- benzimidazol-2-yl] carbamate, is a drug developed for the treatment of human and veterinary helminthic infections. It has been approved by the US Food and Drug Administration (FDA) and is available as generic drug for treating roundworm, common hookworm, American hookworm, pinworm, and whipworm. The closely related albendazole has been approved for treating many parasites in the central nervous system (CNS). Both have been shown to cross the blood-brain barrier in sufficient amounts and to act by binding to tubulin subunits preventing its polymerization and thereby causing ultrastructural changes and preventing parasite growth (Bai et al. 2015; Bai et al. 2011; Aleyasin et al. 2015).

Bai et al. have shown that mebendazole (MBZ) and albendazole (ABZ) can attenuate glioblastoma in rodent models in vivo and in vitro. The study included testing the agents on in vitro human cell lines and tissue culture, measuring cell growth, but also animal experiments in vivo using cell lines transplanted intracerebrally. Mean survival was shifted from 20 to 49 and 36 days after treatments with MBZ or ABZ respectively. Both have been shown to have some efficacy in various experimental forms of cancers (Sandström et al. 1994; Sun et al. 2016; De Witt et al. 2017).

The taxanes are known as chemotherapy agents and widely used. We can cite paclitaxel (Taxol^{®}) and docetaxel (Taxotere^{®}) but also cabazitaxel approved to treat hormone-refractory prostate cancer. The vinca alkaloids are also known as chemotherapy agents. To date the four major vinca alkaloids in clinical use for fighting cancer are vinblastine, vinorelbine, vincristine and vindesine. The epothilones are a new class of chemotherapy agents with regards to the taxanes and the vinca alkaloids. The compounds are names epothilones A to F and some analogs (such as ixabepilone) are approved for use in the treatment of cancer or undergoing the approbation process.

In the objective of treating glioblastoma the inventors decided to deal with several important issues in parallel. Survival time to glioblastoma after failure of the classical Avastin/Temodal and radiotherapy is limited to few months. Over 600 clinical trials using pharmaceutical agents have been made to attenuate glioblastoma and increase survival, unfortunately without success. The main reasons are that many of these trials rely on agents used to treat other types of cancer not considering the uniqueness of brain networks in terms of plasticity and volume organization. To propagate across the environment of the tumour, cells must strongly reduce their volume to adapt to the restricted space available. This is achieved by ionic changes - notably chloride, sodium and water and cytoskeletal contraction. Our suggestion is to combine both approaches to enhance efficacy by preventing these changes to take place, notably volume and cytoskeletal changes and at the same time facilitate apoptosis and hence reduction of the tumour.

Despite the beneficial activities NKCC1 protein inhibitors and microtubules inhibitors, there is a continuing need for new compounds and therapies to treat the aforementioned diseases and conditions. Relying on the well demonstrated experimental data validating different effects and mechanisms of action of the two families of compounds, NKCC1 inhibitors acting on chloride and ion transport and tubulin polymerization inhibitors acting on cytoskeleton without interference with ions channels; we decided to test the possibility of combination of two compounds that might enhance their respective effects and/or show a synergistic effect. This approach has several advantages including reducing the minimal dosages required, decreasing the possible side effects and augmenting the efficacy of both compounds acting on two different mechanisms and possibly exerting a synergistic action on the various toxic elements of tumours and cancers, namely metastasis, diffusion and tumour enhancement, and augmenting at the same time apoptosis.

The present invention describes the combined use of a NKCC1 inhibitor and a microtubule inhibitor for a use in therapy. It particularly describes a NKCC1 inhibitor and a tubulin polymerization inhibitor for use in treating a tumour and/or a cancer in a subject in need thereof, said use comprising administering to a subject at least one NKCC1 inhibitor and at least one tubulin polymerization inhibitor. The combined administration to a subject of a NKCC1 inhibitor and a microtubule inhibitor is used for the treatment of tumours and cancers and particularly to attenuate glioblastoma and related severe tumours.

In the present invention the term "NKCC 1 inhibitor", single or plural, refers to compounds able to inhibit partially or totally the activity of the protein NKCC1. The proteins NKCC1 are cation-chloride cotransporters (CCCs) and secondary active symporters that control the K⁺ and/or Na⁺ gradients to extrude or uptake Cl⁻ in various cell types. Such NKCC inhibitors are selected from sodium dichloroacetate, bumetanide, furosemide, azosemide, piretanide, torasemide, tripamide and their derivatives such as salts and prodrugs. To date, bumetanide is a well-known specific inhibitor of NKCC1 that has already been used in clinical studies (FDA and EP approved) so bumetanide is a preferred NKCC1 inhibitor according to the present invention. According to the invention the NKCC1 inhibitor is selected from sodium dichloroacetate, bumetanide, furosemide, azosemide, piretanide, torasemide, tripamide and their derivatives such as salts and prodrugs.

The term "salt", singular or plural, refers to a pharmaceutically acceptable salts that are salts with inorganic or organic acids, e.g. hydrochloric, hydrobromic, nitric, carbonic, formic, monohydrogencarbonic, phosphoric, monohydrogenphosphoric, dihydrogenphosphoric, perchloric, sulfuric, monohydrogensulfuric, hydroiodic, phosphorous, acetic, lactic, propionic, butyric, isobutyric, palmoic, maleic, glutamic, hydroxymaleic, malonic, benzoic, succinic, glycolic, suberic, fumaric, mandelic, phthalic, salicylic, benzenesulfonic, p-tolylsulfonic, citric, tartaric, methanesulfonic and hydroxynaphthoic acids. The term "salt" can also refer to salts with inorganic bases, e.g. alkali metal bases, especially sodium or potassium bases or alkaline-earth metal bases, especially calcium or magnesium bases, or with pharmaceutically acceptable organic bases.

The term "prodrug" refers to a pharmaceutically acceptable prodrug of the NKCC1 inhibitors are derivatives which have chemically or metabolically cleavable groups and become, by solvolysis or under physiological conditions, the NKCC1 inhibitors which are pharmaceutically active in vivo. Prodrugs of the NKCC1 inhibitors may be formed in a conventional manner with a functional group. The prodrug derivative form often offers advantages of solubility, tissue compatibility or delayed release in a mammalian organism (see, Bundgaard, H., Design of Prodrugs, pp. 7-9, 21-24, Elsevier, Amsterdam 1985). Prodrugs include acid derivatives well known to the person skilled in the art, such as, for example, esters prepared by reaction of the parent acidic compound with a suitable alcohol, or amides prepared by reaction of the parent acid compound with a suitable amine.

According to the invention the terms "microtubules inhibitors" or "tubulin polymerization inhibitors", single or plural, are equivalent and refer to a family of compounds able to stabilize or destabilize microtubule, thereby suppressing microtubule dynamics required for proper mitotic functions, effectively blocking cell cycle progression and resulting in apoptosis. The microtubule inhibitor is selected from the anthelmintics benzimidazole group, the taxanes, the vinca alkaloids and the epothilones. The taxanes includes paclitaxel, docetaxel and cabazitaxel. The vinca alkaloids include vinblastine, vinorelbine, vincristine and vindesine. The epothilones include epothilones A to F and their analogs such as ixabepilone).

In the present invention the term "anthelmintic" drug or compound is the term used to describe a compound or drug used to treat infections of animals or humans with parasitic worms; said worms include both flat worms, i.e. trematodes and cestodes as well as round worms also called nematodes. The term "anthelmintics benzimidazole" or "anthelmintics benzimidazole group" or "benzimidazole compounds having an anthelmintics activity" refers to a group of compounds with a benzimidazole group and displaying anthelminthic activity including mebendazole, albendazole, flubendazole, parbendazole, oxibendazole, oxfendazole, ricobendazole, fenbendazole, triclabendazole, thiabendazole, cambendazole and nocodazole. The combined administration according to the invention is based on the fact that the two compounds can act on different sites: chloride and sodium regulation for the NKCC1 inhibitors and the cytoskeleton for the microtubule inhibitor. The combined administration has an effect on two processes that are essential for the metastatic activity observed in tumour development and cancer. Said processes are the cellular volume modification and the cytoskeleton contraction. The effect of the combined administration on tumours and/or cancer, and particularly on glioblastoma and related severe brain tumours, is higher than the efficacy of each compound alone; there is a synergistic effect of the combined administration according to the results obtained in vitro and in vivo.

Prior art experimental data suggests the use of bumetanide and to a much lesser extent mebendazole, can attenuate glioblastoma in vitro and in vivo, but the combined administration of bumetanide as a NKCC1 inhibitor and mebendazole as a microtubule inhibitor, has never been explored. The present invention provides preclinical evidences and a pilot clinical trial data validating a significative synergistic effect of the combined administration of a NKCC1 inhibitor and a microtubule inhibitor, in particular of bumetanide and mebendazole.

The characteristics of the invention, as well as others, will more clearly appear on reading the following description of an example of realization, said description referring to the attached figures, among which:

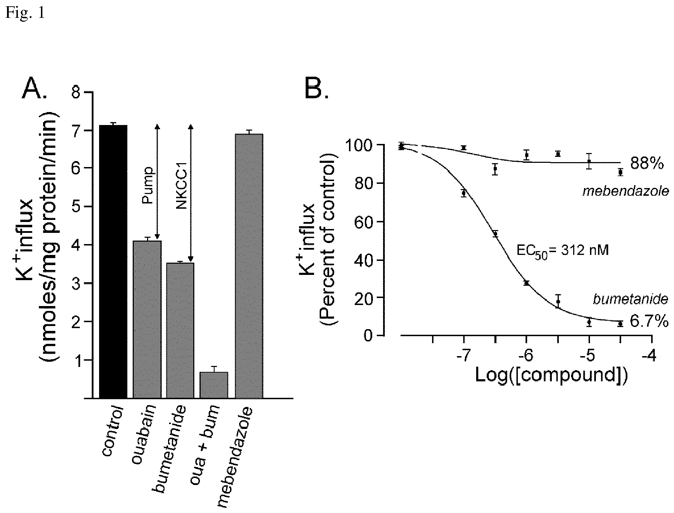

Fig. 1 shows the mechanisms of action of mebendazole, an anthelmintic benzimidazole as a microtubule inhibitor and bumetanide as a NKCC1 inhibitor on HEK293 cells. The Fig. 1A shows the different mechanisms of action of each compound and the Fig. 1B shows the efficacy of bumetanide in inhibiting the K⁺ influx instead of mebendazole. This illustrates the effect of each of the combination partners: the NKCC1 inhibitors acts on the chloride and sodium regulation and the microtubule inhibitor does not.

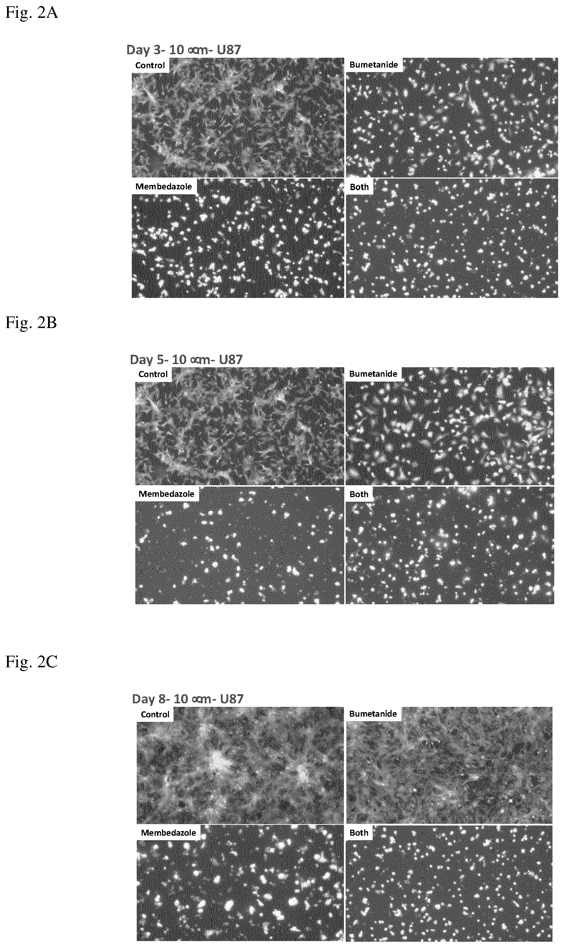

Fig. 2 shows the effects of bumetanide and mebendazole, alone or in combination, in U87 cells at 10µm. The cells are GFP transfected and would appear in green on a coloured figure. Measured were made at day 3 (Fig. 2A), day 5 (Fig. 2B) and day 8 (Fig. 2C) of culture. The dosage of 10µm is a high concentration of each compound and it appears that mebendazole alone exerts a very strong cytotoxic effect and bumetanide also. As observed in subjects having a tumour and/or cancer, after a period of response to treatment the cells show a resistance to chemical therapy, leading to death of the tissue and/or subject. This is illustrated here with each of the combination partners alone at day 8. But with the combined administration of bumetanide and mebendazole, it seems that the emergence of resisting cells is neutralized.

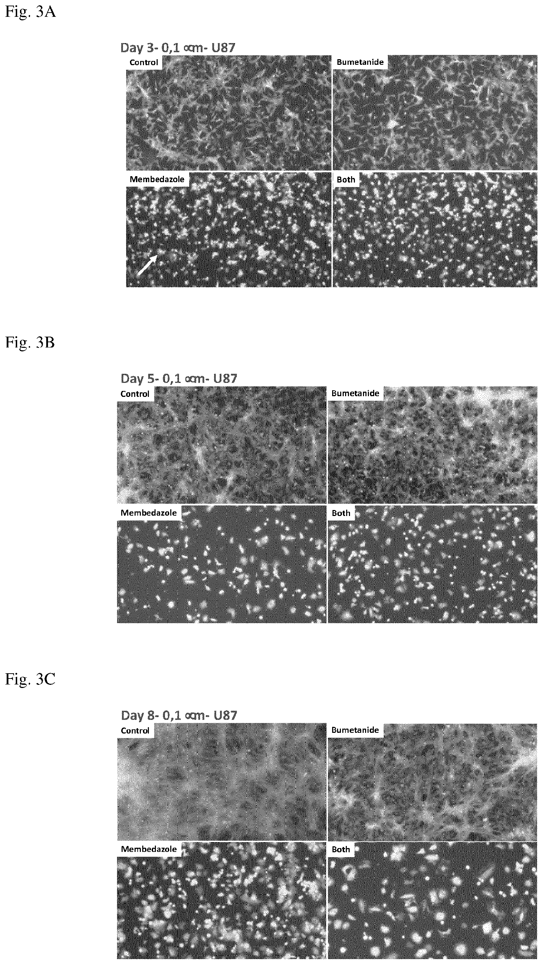

Fig. 3 shows the effects of bumetanide and mebendazole, alone or in combination, in U87 cells at 0.1µm. This is a dosage that was poorly explored in the prior art for the combination partners alone. Measured were made at day 3 (Fig. 3A), day 5 (Fig. 3B) and day 8 (Fig. 3C) of culture. The same results as with the higher dose detailed above are observed. It appears that mebendazole alone exerts a very strong cytotoxic effect at this low dosage. The combined administration according to the invention neutralizes the emergence of resisting cells at day 8.

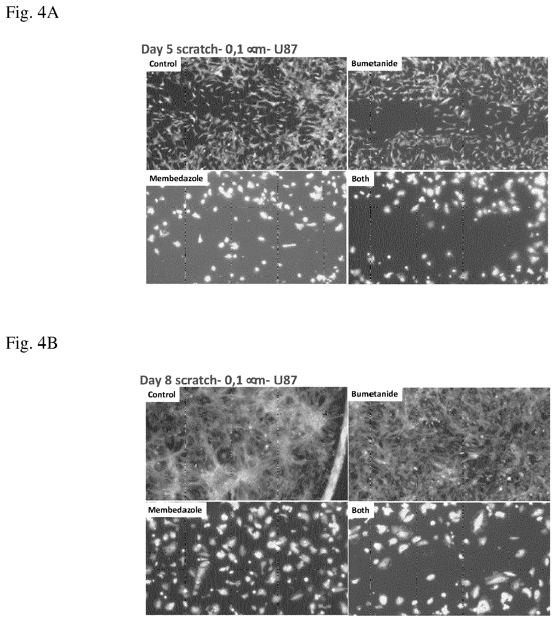

Fig. 4 shows a scratch test on U87 cells at day 5 (Fig. 4A) and at day 8 (Fig. 4B), at 0.1µm of bumetanide, mebendazole and the combination of bumetanide and mebendazole. The combined partners alone have no anti-invasive action at day 5 or at day 8. But the combined administration of the combination partners has a significative anti-invasive action illustrated by the trench on the Fig. 4A and 4B; moreover, a significantly higher number of dead cells with pycnotic and fragmented nuclei are observed.

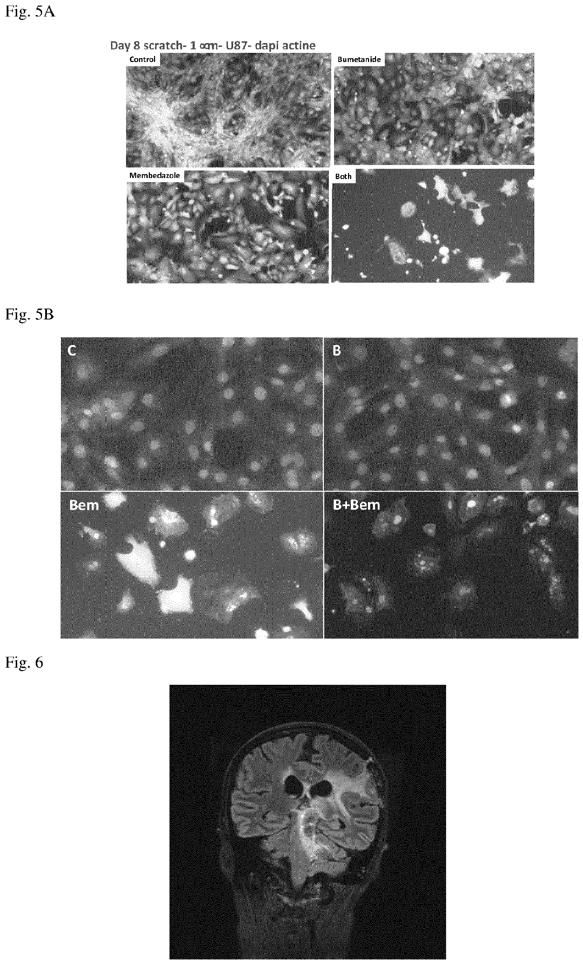

Fig. 5 shows the effects of bumetanide and mebendazole in an immunochemistry test on U87 cells, alone or in combination, at 1µm. The labels used are Dapi (blue) iodure propidium (red) and Actin immunocytochemistry with GFP (green) in order to visualize the viability and non-viability of cells. The Fig. 5A confirms that with low dosages of either bumetanide (B) or mebendazole (Bem) alone, cells are viable staining with both GFP (green) and propidium iodine (red). In contrast, with combined use of both agents, most cells are dead with a considerable reduction of cell density validating their synergistic actions on diffusion and metastasis. This is also shown in Fig. 5B with higher magnification to illustrate with more details the alive and dying cells.
Fig. 6 shows a MRI of a patient (compassionate use single case) with a glioblastoma and especially its brain stem after a combined administration of bumetanide and mebendazole for several months.

The term "combination" refers to either a fixed combination in one dosage unit form, or a non- fixed combination (or kit of parts) for the combined administration where a NKCC1 inhibitor and a microtubule inhibitor may be administered independently at the same time or separately within time intervals, especially where these time intervals allow that the combination partners show a cooperative, i.e. synergistic effect.

As used herein the term "combination partners" or "combination compounds" or "combined therapeutic agents" are equivalent and refer to at least one NKCC1 inhibitor and at least one microtubule inhibitor.

The term "combined administration" or the like as utilized herein are meant to encompass administration of the selected combination partner to a single subject in need thereof (i.e. a patient), and are intended to include treatment regimens in which the agents are not necessarily administered by the same route of administration or at the same time.

The term "fixed combination" means that the active ingredients, i.e. a NKCC1 antagonist and a microtubule inhibitor, are both administered to a patient simultaneously in the form of a single entity or dosage. The terms "non-fixed combination" or "kit of parts" mean that the active ingredients, i.e. a NKCC1 inhibitor and a microtubule inhibitor, are both administered to a patient as separate entities either simultaneously, concurrently or sequentially with no specific time limits, wherein such administration provides therapeutically effective levels of the two compounds in the body of the subject in need thereof.

The term "treatment" includes prophylactic and therapeutic treatment (including but not limited to palliative, curing, symptom-alleviating, symptom-reducing) as well as the delay of progression of a cancer disease or disorder. The term "prophylactic" means the prevention of the onset or recurrence of a cancer. The term "delay of progression" as used herein means administration of the combination to patients being in a pre-stage or in an early phase of the cancer to be treated, a pre-form of the corresponding cancer is diagnosed and/or in a patient diagnosed with a condition under which it is likely that a corresponding cancer will develop. "Pharmaceutical preparation" or "pharmaceutical composition" refers to a mixture or solution containing at least one therapeutic agent to be administered to a patient, i.e., a human. "Pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio. "Therapeutically effective" preferably relates to an amount of a therapeutic agent that is therapeutically or in a broader sense also prophylactically effective against the progression of a tumour and/or cancer.

The terms "medicament" or "drug" or "therapeutic agent" are considered and used herein as equivalents and refer to one of the combination partners alone or both combination partners. "Jointly therapeutically effective" means that the therapeutic agents may be given separately (in a chronologically staggered manner, especially a sequence-specific manner) in such time intervals that they prefer, in the warm-blooded animal, especially human, to be treated, still show a (preferably synergistic) interaction. Whether this is the case can, inter alia, be determined by following the blood levels, showing that both compounds are present in the blood of the human to be treated at least during certain time intervals.

In one embodiment the combination partners further include at least one pharmaceutically acceptable carrier material. Said pharmaceutically acceptable carrier material refers to a single carrier or vehicle formulated to deliver effective amounts of both combination partners to a subject in need thereof. The single vehicle is designed to deliver an effective amount of each of the compounds, along with any pharmaceutically acceptable carriers or excipients. In some embodiments, the vehicle is a tablet, capsule, pill, or a patch. In other embodiments, the vehicle is a solution or a suspension.

The term "patient" or "subject" refers to a human or other animal, preferably a human, in need of a treatment according to the invention.

The present invention relates to a therapeutic combination useful in therapy and especially useful for the treatment of cancer. In particular, the invention relates to a therapeutic combination that comprises at least one NKCC1 protein inhibitor and at least one microtubule inhibitor, optionally together with one or more additional chemotherapeutic agents or radiotherapy.

The present invention describes a NKCC1 protein inhibitor and a microtubule inhibitor for use in therapy. It especially describes a NKCC1 protein inhibitor and a microtubule inhibitor for use in treating a cancer in a subject in need thereof, comprising administering to the subject at least one NKCC1 inhibitor and at least one microtubule inhibitor as combination partners. The at least one NKCC1 inhibitor and at least one microtubule inhibitor are also referred to as combination partners or as compounds or drugs.

The invention further describes the compounds for use as detailed above, for simultaneous, separate or sequential use of the combination partners so that they are jointly active. Said compounds for use are selected as follows:
- the microtubule inhibitor is selected from benzimidazole anthelmintics, taxanes, vinca alkaloids and epothilones; and the benzimidazole anthelmintic is selected from mebendazole, albendazole, flubendazole, parbendazole, oxibendazole, oxfendazole, ricobendazole, fenbendazole, triclabendazole, thiabendazole, cambendazole and nocodazole.
- the NKCC1 protein inhibitor is selected from sodium dichloroacetate, bumetanide, furosemide, azosemide, piretanide, torasemide, tripamide and their derivatives such as salts and prodrugs.

In a preferred embodiment the NKCC1 protein inhibitor is bumetanide. In another preferred embodiment the combination partners are selected from bumetanide as the first partner and mebendazole or albendazole as the second partner. In another embodiment the NKCC 1 inhibitor is bumetanide and the microtubule inhibitor is selected from mebendazole and albendazole.

The combined administration of at least one NKCC1 inhibitor and at least one microtubule inhibitor for the treatment of a tumour and/or cancer is particularly useful for the treatment of a mastocytoma or a mast cell tumour, an ovarian cancer, a pancreatic cancer, a non-small cell lung cancer, a small cell lung cancer, hepatocarcinoma, a melanoma, a retinoblastoma, a breast tumour, a colorectal carcinoma, a leukaemia, a lymphoma, an acute lymphoblastic leukaemia or acute lymphoid leukaemia, an acute myeloid leukaemia, a histiocytic sarcoma, a brain tumour, an astrocytoma, a glioblastoma, a neuroma, a neuroblastoma, a colon carcinoma, a cervical carcinoma, a sarcoma, a prostate tumour, a bladder tumour, a tumour of the reticuloendothelial tissues, a Wilm's tumour, an ovarian carcinoma, a bone cancer, an osteosarcoma, a renal cancer, or head and neck cancer, an oral cancer, a laryngeal cancer, or an oropharyngeal cancer. In a preferred embodiment the combined administration is useful for the treatment of glioblastoma and severe related tumours.

In one embodiment the combined administration of a NKCC1 inhibitor and a microtubule inhibitor further comprises any one of chemotherapy, radiotherapy or chemoradiotherapy.

In a further embodiment the combination partners are use in the preparation of a medicament for the prevention, delay or treatment of a tumour and/or cancer. Said medicament or drug is particularly useful for the treatment of a mastocytoma or a mast cell tumour, an ovarian cancer, a pancreatic cancer, a non-small cell lung cancer, a small cell lung cancer, hepatocarcinoma, a melanoma, a retinoblastoma, a breast tumour, a colorectal carcinoma, a leukaemia, a lymphoma, an acute lymphoblastic leukaemia or acute lymphoid leukaemia, an acute myeloid leukaemia, a histiocytic sarcoma, a brain tumour, an astrocytoma, a glioblastoma, a neuroma, a neuroblastoma, a colon carcinoma, a cervical carcinoma, a sarcoma, a prostate tumour, a bladder tumour, a tumour of the reticuloendothelial tissues, a Wilm's tumour, an ovarian carcinoma, a bone cancer, an osteosarcoma, a renal cancer, or head and neck cancer, an oral cancer, a laryngeal cancer, or an oropharyngeal cancer. In a preferred embodiment the medicament or drug is useful for the treatment of glioblastoma and severe related tumours.

In one embodiment of the invention the therapeutic combination is formulated as separate compositions (one for each combination partner or therapeutic agent), or the two partners of the therapeutic combination are formulated into one composition or drug formulation (the two combination partners are formulated together).

In another embodiment of the invention the combination partners, i.e. the NKCC1 inhibitor and the microtubule inhibitor, are packaged individually, or are packaged together, or packaged in any combination, in a single package, a plurality of packages or packettes, or a blister packet, lidded blister or blister card or packets, or a shrink wrap.

In another embodiment one or the two drugs of the therapeutic combination are formulated or manufactured as a parenteral formulation, an aqueous solution, a liposome, an injectable solution, a tablet, a pill, a lozenge, a capsule, a caplet, a patch, a spray, an inhalant, a powder, a freeze-dried powder, an inhalant, a patch, a gel, a geltab, a nanosuspension, a nanoparticle, a nanoliposome, a microgel, a pellet, a suppository or any combination thereof.

In a particular embodiment the dosage of the NKCC1 inhibitor ranges from 1 to 5 mg a day for an adult patient. The dosage of the microtubule inhibitor ranges from 500 mg to 1500 mg a day for an adult patient. In another embodiment both compounds are packaged in dosages that match a chrono-dosing regimen to match an optimal dose for the time of day. The term "Dose range" refers to an upper and a lower limit of an acceptable variation of the amount of combination partner specified. Typically, a dose of the combination partners in any amount within the specified range can be administered to a subject undergoing treatment.

The therapeutic combination of the invention is formulated for administration intravenously, topically, orally, by inhalation, by infusion, by injection, by inhalation, intraperitoneally, intramuscularly, subcutaneously, intra-aurally, for intra-articular administration, for intra-mammary administration, for topical administration or for absorption through epithelial or mucocutaneous linings.

Such combination may be for simultaneous, separate or sequential use for the treatment of a tumour and/or a cancer.

In alternative embodiments, provided are a device, a medical device, an implant, a breast implant, a prosthesis, a stent, a catheter, comprising a therapeutic combination of therapeutic agents or partners as provided herein.

In a further embodiment the invention provides a pharmaceutical composition comprising at least one NKCC1 inhibitor, at least one microtubule inhibitor and a pharmaceutically acceptable excipient or adjuvant, for use in therapy, and especially for use in treating a tumour and/or cancer in a subject in need thereof. The present invention provides a pharmaceutical composition comprising the therapeutic combination as provided herein, and optionally the pharmaceutical composition can further comprise a pharmaceutically acceptable excipient. In one embodiment, the pharmaceutical composition is formulated or manufactured as a feed, a food, a food or feed concentrate, a pellet, a lozenge, a liquid, a lotion, an implant, a nanoparticle, an elixir, an aerosol, a spray, an aerosol, an inhalant, a powder, a tablet, a pill, a capsule, a gel, a geltab, a nanosuspension, a nanoparticle, a patch, a microgel or a suppository.

In alternative embodiments, provided are methods for treating, preventing or ameliorating a tumour and/or a cancer, comprising: applying or administering to an individual in need thereof; or, applying or administering to an effected tissue: the therapeutic combinations as provided herein, or a pharmaceutical composition as provided herein, wherein optionally the therapeutic agents or drugs are administered separately or together, or at the same time, or in synchrony, or by chrono-dosing, or one of the therapeutic agents or drugs is administered before another of the therapeutic agents or drugs, and optionally the therapeutic agents or drugs are formulated for administration intravenously (IV), parenterally, nasally, topically or locally, orally, or by liposome, implant or vessel-targeted nanoparticle delivery.

According to the invention the cancer or tumour is: a mastocytoma or a mast cell tumour, an ovarian cancer, pancreatic cancer, a non-small cell lung cancer, small cell lung cancer, hepatocarcinoma, melanoma, retinoblastoma, breast tumour, colorectal carcinoma, leukaemia, lymphoma, acute lymphoblastic leukaemia or acute lymphoid leukaemia, acute myeloid leukaemia, a histiocytic sarcoma, a brain tumour, an astrocytoma, a glioblastoma, a neuroma, a neuroblastoma, a colon carcinoma, cervical carcinoma, sarcoma, prostate tumour, bladder tumour, tumour of the reticuloendothelial tissues, Wilm's tumour, ovarian carcinoma, a bone cancer, an osteosarcoma, a renal cancer, or head and neck cancer, oral cancer, a laryngeal cancer, or an oropharyngeal cancer.

The invention further provides methods for treating, preventing or ameliorating a tumour or a cancer, comprising: applying or administering to an individual in need thereof; or, applying or administering to an effected tissue: the therapeutic combinations as provided herein, or a pharmaceutical composition or formulation as provided herein, wherein optionally the therapeutic agents or drugs are administered separately or together, or at the same time, or in synchrony, or by chrono-dosing, or one of the therapeutic agents or drugs is administered before the other of the therapeutic agents or drugs, and optionally the therapeutic agents or drugs are formulated for administration intravenously, parenterally, nasally, topically or locally, orally, or by liposome, implant or vessel-targeted nanoparticle delivery.

In one embodiment the methods further comprise an anticancer or anti-tumour radiotherapy or a proton beam therapy.

In another embodiment, provided are uses of a therapeutic combination as provided herein in the manufacture of a medicament. In a particular embodiment, provided are uses of the therapeutic combinations as provided herein, in the manufacture of a medicament for treating a cancer or a tumour; said cancer or tumour is selected from a mastocytoma or a mast cell tumour, an ovarian cancer, pancreatic cancer, a non-small cell lung cancer, small cell lung cancer, hepatocarcinoma, melanoma, retinoblastoma, breast tumour, colorectal carcinoma, leukaemia, lymphoma, acute lymphoblastic leukaemia or acute lymphoid leukaemia, acute myeloid leukaemia, a Histiocytic sarcoma, a brain tumour, an astrocytoma, a glioblastoma, a neuroma, a neuroblastoma, a colon carcinoma, cervical carcinoma, sarcoma, prostate tumour, bladder tumour, tumour of the reticuloendothelial tissues, Wilm's tumour, ovarian carcinoma, a bone cancer, an osteosarcoma, a renal cancer, or head and neck cancer, oral cancer, a laryngeal cancer, or an oropharyngeal cancer.

The invention will now be described by reference to the following examples and figures, which are merely illustrative and are not to be construed as a limitation of the scope of the present invention.

The present invention describes a NKCC1 inhibitor and a microtubule inhibitor for a use in therapy, or their use in the preparation of a medicament or the pharmaceutical composition containing them as combination partners for their use in therapy. Said therapy is preferentially a tumour and/or cancer therapy. In a preferred embodiment the invention describes a NKCC1 inhibitor and a microtubule inhibitor, or their use in the preparation of a medicament or the pharmaceutical composition containing them as combination partners for their use in tumour and/or cancer therapy wherein the tumour and/or cancer is selected from a mastocytoma or a mast cell tumour, an ovarian cancer, a pancreatic cancer, a non-small cell lung cancer, a small cell lung cancer, hepatocarcinoma, a melanoma, a retinoblastoma, a breast tumour, a colorectal carcinoma, a leukaemia, a lymphoma, an acute lymphoblastic leukaemia or acute lymphoid leukaemia, an acute myeloid leukaemia, a histiocytic sarcoma, a brain tumour, an astrocytoma, a glioblastoma, a neuroma, a neuroblastoma, a colon carcinoma, a cervical carcinoma, a sarcoma, a prostate tumour, a bladder tumour, a tumour of the reticuloendothelial tissues, a Wilm's tumour, an ovarian carcinoma, a bone cancer, an osteosarcoma, a renal cancer, or head and neck cancer, an oral cancer, a laryngeal cancer, or an oropharyngeal cancer. In another preferred embodiment the invention describes a NKCC 1 inhibitor and a microtubule inhibitor, or their use in the preparation of a medicament or the pharmaceutical composition containing them as combination partners for their use in the treatment of glioblastoma and severe related tumours.

### EXAMPLES

The following studies were made on mebendazole or albendazole as microtubules inhibitors and bumetanide as NKCC1 inhibitor.

The HEK cell line is a specific immortalised cell line widely used in cell biology and biotechnology research and the U87 cell line is a human glioblastoma cell line that is commonly used in brain cancer research.
1) Validation of the mechanisms of action of mebendazole and bumetanide Using the human HEK293 cell line, we confirm that bumetanide but not mebendazole act on NKCC1 altering potassium transport. As shown in Figure 1A, K⁺ influx in HEK293 cells is inhibited 44% by 100 uM ouabain, whereas the flux is inhibited 49% by bumetanide. Combined, the two drugs reduce K⁺ influx by 90%. The remaining 10% is of the flux is mediated by K⁺ channels. The Figure also shows that mebendazole is ineffective in inhibiting K⁺ influx. Figure 1B reports dose response curves for bumetanide and mebendazole on NKCC1-mediated K⁺ influx, showing efficacy of bumetanide (EC₅₀ = 312 nM) and inefficacy of mebendazole. These data show that mebendazole does not affect the Na⁺/K⁺-pump, nor the Na-K-2Cl cotransporter.
2) Effect of the combined use of bumetanide and mebendazole

The effects of bumetanide alone or in combination with mebendazole were investigated in U87 cell lines at 0.1, 1, and 10µm. Each experiment was performed in triplicate (n=3). Cells were GFP transfected (green). Cytotoxicity is manifested by loss of GFP and presence of small round flat cells loosing GFP expression indicative of cell death (apoptosis).

### A) at a dosage of 10µm

The Fig. 2 shows the effect of control, bumetanide, mebendazole and bumetanide + mebendazole at 10 µm on the U87 cell lines at day 3 (Fig. 2A), day 5 (Fig. 2B) and day 8 (Fig. 2C).

As shown in Fig. 2, at the high concentration of 10µm mebendazole (MEB or MEB) exerts a very strong cytotoxic effect (55 % reduction of cell number at day 1, 89% at day 5- p < 0,001, SD 3,1). Interestingly at day 8 the cytotoxic effect decreased to 64 % (p<0,05, SD 2) with the appearance of flat proliferating cell, demonstrating the occurrence of drug-resistance with, as observed in patients, the expression of cells that resist to chemical-therapy leading to death. Bumetanide induces a strong cytotoxic action at day 3 and day 5 but less so at day 8 (non-significant reduction of cell number (18 %)) also suggesting a resistance after long delays. The combination of both drugs neutralized the emergence of these resisting cells. These results suggest that the combined actions of bumetanide and mebendazole are complementary abolishing the resistance of tumor cells to the treatment.

### B) at a dosage of 0.1µm

To further explore the synergistic effect between bumetanide and mebendazole, we decreased the concentration from 10µm to 0.1µm which is a very low concentration poorly explored in the literature.

The results are shown in Fig. 3 were the Fig. 3A shows the fluorescence at day 3, Fig. 3B shows the fluorescence at day 5 and Fig. 3C shows the fluorescence at day 8.

At day 3, the number of cells was reduced significantly (43 % (p<0,001, SD 1,8) by mebendazole but not by bumetanide. It seems that the combination of both drugs did not enhance the effects of mebendazole alone.

At day 5, the number of cells was reduced by 90 % (p<0,001, SD 2,9) by mebendazole but not by bumetanide and there was no synergy when both drugs were combined.

In contrast, at day 8, the effects of mebendazole alone were reduced (70 %) and the appearance of round flat cells indicated cell death. When both drugs are combined, the number of remaining cells was reduced by 90% (p<0;001, SD 1,5) indicating that the above detailed resistance was neutralized.

This validates the results obtained with higher dosage but also show a clear synergistic effect at a very low dosage.

### 3) Scratch test

To further investigate invasion of tumour cells, we performed a scratch test. This test enables to visualize the propagation and metastasis of tumour cells determined by their capacity to cross the trench. Therefore, U87 cells are cultivated and a scratch is made with a blade in order to estimate the strength and rapidity of invasion.

Fig. 4A shows the tumour cells at day 5 at 0.1µm and the Fig. 4B shows the tumour cells at day 8 at 0.1µm.

As shown in Figure 4A, bumetanide reduced significantly invasion by 40 % at day 5 (p<0,001; SD 1,3) when compared to controls who were not treated. Mebendazole failed to reduce significantly cell invasion (15%). In contrast, applications of bumetanide and mebendazole reduced invasion by 95% (p<0,001; SD 0,8).

At day 8 (Fig. 4B), neither applications of mebendazole or bumetanide separately produced an anti-invasive action. However, at Day 8, associating bumetanide with mebendazole rescued this failure by 82% (p<0,001; SD 1,1) visualized by the persistence of a trench. Also, in contrast to separate applications of bumetanide or mebendazole, a significantly higher number of dead cells with pycnotic and fragmented nuclei was observed with a combined administration of mebendazole and bumetanide.

The conclusion of the scratch study is that combined administration of bumetanide and mebendazole at low doses that are inefficient separately, prevents the propagation and metastasis of tumour cells validating their synergistic action.

### 4) Immunochemistry

To better illustrate the effects of bumetanide and mebendazole, we used Dapi (blue) iodure propidium (red) and Actin immunocytochemistry with GFP (green) in order to visualize the viability and non-viability of cells.

The Fig. 5A clearly confirms what was illustrated above using Dapi stain, showing that with low dosages of either bumetanide (B) or mebendazole (Bem) alone, cells are viable staining with both GFP (green) and propidium iodine (red). In contrast, with combined use of both agents, most cells are dead with a considerable reduction of cell density (control: 3%, B: 3,2 %, MEB: 50%, B+MEB: 90%) validating their synergistic actions on diffusion and metastasis. This is also shown in Fig. 5B with higher magnification to illustrate with more details the alive and dying cells.

The combined use of bumetanide and mebendazole clearly enhances the efficacy on both cell death and invasion/metastasis.

### 5) Pilot clinical trial

In the context of a pilot compassionate use one patient with multiples brain metastasis was treated by combined administration of bumetanide (1 mg/day) and mebendazole (Ig/day). Clinical symptoms of multiples brain metastasis were characterized for this patient by violent headaches and left hemiparesis. Magnetic Resonance Imaging (MRI) individualized several brain metastases notably the brain stem (Fig. 6).

Under the combined administration of bumetanide and mebendazole, symptoms massively ameliorated with headaches disappearance and hemiparesis decreased. MRI at month 3 showed necrosis inside the main tumour with no size modification. A significant improvement was observed for 4 months. At month 4 the mebendazole administration was stopped. Neurological symptoms recurred at 5 months with the death of the patient occurring at 8 months.

This compassionate use single case suggests that the combined use of a NKCC1 protein inhibitor, namely bumetanide, and a microtubule inhibitor, namely mebendazole, can produce a significant improvement in one patient with very aggressive brain metastasis.

The above results show for the first time that the actions of mebendazole and bumetanide are complementary or synergistically acting on different mechanisms that converge in order to enhance the reactivity of the targeted tissue. The combined administration reduces efficiently the propagation of the tumour with low doses of each of the combination partners; said doses having little or no action separately on a glioblastoma cell line, do attenuate the symptoms when combined.

The results were confirmed in tumoroids freshly dissected and placed in a chamber. The combination partners act accordingly, validating the synergistic effect of the combination.

The above results demonstrate the synergistic impact of a combination of bumetanide and mebendazole in neutralizing cytotoxic resistance and increasing the anti-invasive effect with the induction of a strong cytotoxic/apoptotic effect. This significative effect is observed at very low doses of each of the combination partners, doses that will have very limited side effects in clinical conditions.

This is a very promising treatment of tumours and/or cancer in subjects in need thereof.

## Claims

1. A NKCC1 protein inhibitor and a microtubule inhibitor for use in therapy.

2. A NKCC1 protein inhibitor and a microtubule inhibitor for use in treating a cancer in a subject in need thereof, comprising administering to the subject at least one NKCC1 inhibitor and at least one microtubule inhibitor as combination partners.

3. The compounds for use according to claim 1 or 2, for simultaneous, separate or sequential use of the combination partners so that they are jointly active.

4. The compounds for use according to any of claims 1 to 3, wherein the microtubule inhibitor is selected from benzimidazole anthelmintics, taxanes, vinca alkaloids and epothilones.

5. The compounds for use according to any of claim 3, wherein the benzimidazole anthelmintic is selected from mebendazole, albendazole, flubendazole, parbendazole, oxibendazole, oxfendazole, ricobendazole, fenbendazole, triclabendazole, thiabendazole, cambendazole and nocodazole.

6. The compounds for use according to any of claims 1 to 4, wherein the NKCC1 protein inhibitor is selected from sodium dichloroacetate, bumetanide, furosemide, azosemide, piretanide, torasemide, tripamide and their derivatives.

7. The compounds for use according to any of claims 1 to 6, wherein the NKCC1 protein inhibitor is bumetanide.

8. The compounds for use according to any one of claims 1 to 7 wherein the microtubule inhibitor is selected from mebendazole and albendazole.

9. The compounds for use according to any of claims 1 to 8, further including at least one pharmaceutically acceptable carrier material.

10. The compounds for use according to any of claims 1 or 9, wherein the use further comprises any one of chemotherapy, radiotherapy or chemoradiotherapy.

11. The use of the compounds for use according to any one of claims 1 to 10 in the preparation of a medicament for prevention, delay or treatment of tumour and/or cancer.

12. A pharmaceutical composition comprising at least one NKCC1 inhibitor, at least one microtubule inhibitor and a pharmaceutically acceptable excipient or adjuvant, for use in therapy.

13. The pharmaceutical composition according to claim 12 for use in treating a tumour and/or cancer in a subject in need thereof.

14. The compounds for use according to any of claims 1 to 10 or the use according to claim 11 or the pharmaceutical composition of claims 12 and 13 wherein the tumour and/or cancer is selected from a mastocytoma or a mast cell tumour, an ovarian cancer, a pancreatic cancer, a non-small cell lung cancer, a small cell lung cancer, hepatocarcinoma, a melanoma, a retinoblastoma, a breast tumour, a colorectal carcinoma, a leukaemia, a lymphoma, an acute lymphoblastic leukaemia or acute lymphoid leukaemia, an acute myeloid leukaemia, a histiocytic sarcoma, a brain tumour, an astrocytoma, a glioblastoma, a neuroma, a neuroblastoma, a colon carcinoma, a cervical carcinoma, a sarcoma, a prostate tumour, a bladder tumour, a tumour of the reticuloendothelial tissues, a Wilm's tumour, an ovarian carcinoma, a bone cancer, an osteosarcoma, a renal cancer, or head and neck cancer, an oral cancer, a laryngeal cancer, or an oropharyngeal cancer.

15. The compounds for use according to any of claims 1 to 10 or the use according to claim 11 or the pharmaceutical composition of claims 12 and 13 wherein the tumour and/or cancer is glioblastoma and severe related tumours.
